# EUROPEAN PATENT SPECIFICATION

(11) **EP 4 267 960 B1**
(45) Date of publication and mention of the grant of the patent: **25.06.2025**
(21) Application number: 21911752.0
(22) Date of filing: 24.12.2021
(51) Int. Cl.: G01N 33/53

(54) **METHODS USING ANTI-SLC3A2 AUTOANTIBODIES AND SLC3A2 FOR DETECTION OF GLIOMA BRAIN TUMOR FROM PATIENT SERUM**
VERFAHREN MIT ANTI-SLC3A2-AUTOANTIKÖRPERN UND SLC3A2 ZUM NACHWEIS VON GLIOM-HIRNTUMOREN AUS PATIENTENSERUM
PROCÉDÉS UTILISANT DES AUTO-ANTICORPS ANTI-SLC3A2 ET SLC3A2 POUR LA DÉTECTION DE TUMEUR CÉRÉBRALE DE GLIOME À PARTIR DE SÉRUM DE PATIENT

(30) Priority: 25.12.2020 TR 202021768
(43) Date of publication of application: 01.11.2023
(73) Proprietor: Istanbul Medipol Universitesi Teknoloji Transfer Ofisi Anonim Sirketi, 34810 Beykoz/Istanbul (TR); Istanbul Üniversitesi, 34093 Istanbul (TR)
(72) Inventor: KARAKAS , Nihal, 34810 Beykoz/ Istanbul (TR); TOPÇU, Ozan, 34810 Beykoz/ Istanbul (TR); TÜZÜN, Erdem, 34093 Istanbul (TR); TIMIRCI KAHRAMAN, Özlem, 34093 Istanbul (TR); ARAS, Yavuz, 34093 Istanbul (TR); SABANCI, Pulat Akin, 34093 Istanbul (TR)
(74) Representative: Simsek, Meliha Merve
(86) International application number: PCT/TR2021/051501
(87) International publication number: WO 2022/139775

(56) References cited:
- US-A1- 2007 105 133
- CHRISTIAN PHILIPP PALLASCH ET AL: "Autoantibodies against GLEA2 and PHF3 in glioblastoma: Tumor-associated autoantibodies correlated with prolonged survival", INTERNATIONAL JOURNAL OF CANCER, JOHN WILEY & SONS, INC, US, vol. 117, no. 3, 19 May 2005 (2005-05-19), pages 456 - 459, XP071282824, ISSN: 0020-7136, DOI: 10.1002/IJC.20929
- HALLAL, S. ET AL.: "Extracellular vesicles released by glioblastoma cells stimulate normal astrocytes to acquire a tumor-supportive phenotype via p53 and MYC signaling pathways", MOLECULAR NEUROBIOLOGY, vol. 56, no. 6, 2019, pages 4566 - 4581, XP036777371, DOI: 10.1007/s12035-018-1385-1
- LANE ROSEMARY, SIMON THOMAS, VINTU MARIAN, SOLKIN BENJAMIN, KOCH BARBARA, STEWART NICOLAS, BENSTEAD-HUME GRAEME, PEARL FRANCES M. : "Cell-derived extracellular vesicles can be used as a biomarker reservoir for glioblastoma tumor subtyping", COMMUNICATIONS BIOLOGY, vol. 2, no. 1, 1 December 2019 (2019-12-01), XP055954547, DOI: 10.1038/s42003-019-0560-x
- SIMON, T. ET AL.: "Breaking through the glioblastoma micro- environment via extracellular vesicles", ONCOGENE, vol. 39, no. 23, 2020, pages 4477 - 4490, XP037154858, DOI: 10.1038/s41388-020-1308-2
- LANGEN, K. J. ET AL.: "3-[123I] iodo-a-methyl-ltyrosine transport and 4F2 antigen expression in human glioma cells", NUCLEAR MEDICINE AND BIOLOGY, vol. 28, no. 1, 2001, pages 5 - 11, XP086047670, DOI: 10.1016/S0969-8051(00)00178-5

## Description

### Technical Field Related to Invention

The invention relates to a method using autoantibodies that enable early diagnosis and treatment of patients with glioblastoma (GB) and/or advanced glioma brain tumor using patient serum and antigens recognized by these autoantibodies.

### The State of Art Related to the Invention (Prior Art)

With current methods, in order to diagnose glioblastoma (GB), which is one of the advanced gliomas, tumor tissue is surgically removed from the patients and pathological markers for GB are analyzed. Therefore, the diagnosis of the disease can only be made after surgical treatment, that is, in the late period when the tumor becomes aggressive and malignancy increases, and this situation directly reduces the life span of the patient.

The diagnosis of the disease first continues with the examination of MR findings and after the surgical operation, radiotherapy and adjuvant chemotherapy (patients are treated with temozolomide). The surgical operation is planned after the detection of the tumor by cranial magnetic resonance imaging (MRI). However, sometimes the tumor image may also be caused by radionecrosis or other brain pathologies, and unfortunately, MRI alone isinsufficient method for glial tumor diagnosis. Therefore, obtaining information about whether the tumor is a glial tumor and/or its stage before and after MRI may guide surgical treatment (by determining the need for needle biopsy or safe maximum resection) and increase the patient's lifetime and quality. In addition, due to the location of the tumor, it may cause damage such as loss of motor skills and speech disorders after surgical treatment is performed without this information. In conclusion, glioma-specific and/or glioma stage-determining diagnostic systems are of great importance in terms of managing the treatment of this malignant (fatal) disease and increasing the survival time and quality of life of patients.

On the other hand, radiotherapy and chemotherapy treatment, which are the current treatment methods, cannot cure the disease while harming healthy tissues and organs as the tumor is not specific. Under normal conditions, patients can live statistically for an average of 15 months after the diagnosis of GB is made. Current diagnosis and treatment methods are inadequate to prolong this period. Therefore, the diagnosis of advanced glioma brain tumor before surgical operation can significantly improve the treatment process of the disease, and if the components of this system provide glioma-specific biomarkers, there can be a second improvement in the struggling with the disease by developing tumor-specific targeted therapy approaches.

Patent document numbered WO2015200823A1 mentions 33 cell surface proteins identified in glioblastoma (GB) tissue that distinguish GB patients from healthy controls with more than 98% accuracy. However, it has not been reported that any of these proteins is an autoantigen or circulating in the blood stream. These proteins can be used in the diagnosis of GB patients, however as they are not antibodies, they are not expected to circulate in patient serum for a long time.

Autoantibodies are indicative of the reaction of organisms to its own antigens. These antigens can also develop against all cell types or against specific cells of an organ of the body. These antibodies provide information about the basic mechanisms of inflammation and loss of tolerance in patients with autoimmune disease. In many autoimmune diseases, the presence of autoantibodies has been observed without the development of any clinical findings months or years ago. Autoantibodies are markers that also indicate specific clinical findings, disease severity, and degree of progression.

Autoantibodies are used in the diagnosis of many diseases, however there are no diagnosis methods and systems especially using autoantibodies specific to GB disease. In the patent document numbered CN102590511B, it is mentioned that IGFBP-2 autoantibodies, which can be used in the diagnosis of glioma along with various cancers, can be detected and used as a diagnostic kit. Pallash et al. (International Journal of cancer, 2005) describes the relevance of auto-antibodies against GLEA2 for evaluating the probability of having glioblastoma.

SLC (solute carriers) carrier proteins consist of genes that form a 65-membered superfamily. They are mainly involved in molecule/drug delivery and are a frequently referenced research resource in drug resistance studies. A sub-classification is made into SLC families according to what type of molecule delivery they are responsible for, and these families include:
SLC1 family, SLC2 family....SLC65 family. In addition, every family has sub-members. For example, the SLC3 family has 2 members: Such as, SLC3a1 and SLC3a2. In general, in the operating mechanism, they act together with members of the SLC7 family, so most studies usually analyze both together. On the other hand, SLC3a2 (other names are CD98; 4F2) forms a dimer with SLC7a5 for its functionality and is involved in the delivery of amino acids together. CD98 is a transmembrane protein and is found on the cell surface. Therefore, it can be easily targeted with drugs.

There is a need for new therapeutic methods for GB disease, which is quite malignant and has a poor prognosis. However, the priority for treatment is early diagnosis of the disease. For this reason, there is a need for methods that will enable the early diagnosis of the disease, thus prolonging the survival time of the patients.

### Summary and Objects of the Invention

With the invention, advanced stage gliomas can be detected quickly without the need for surgical operation in the early period by using only the blood sample of the patient. In the prior art, there is no autoantibody-based diagnostic and prognostic method or kit that can enable the diagnosis and treatment of GB and/or advanced stage glioma patients.

With the invention, the presence of GB-specific anti-SLC (especially anti-SLC3A2 (solute carrier 3a2) in one example of the invention) autoantibodies is indicated by western blot analyzes following immunoprecipitation and increasing autoantibody levels are associated with GB disease by determining the levels of this autoantibody in GB patients and other glioma patients compared to healthy individuals. Apart from the advantage it will offer by detecting the high grades of the disease, it also provides the opportunity to follow the progression of the glioma stage with the differential diagnosis of tumors in which there is no interaction of this autoantibody with SLC3a2 in low-grade gliomas, especially this may help to diagnoseprogression from grade II to GB. Thus, the invention covers the methods that can provide diagnosis of GB tumor and follow-up of the disease by the presence of SLC3a2-specific autoantibodies in the patient's blood and the determination of SLC3a2 expression levels and modifications.

With the invention, it is aimed to determine the level of anti-SLC3a2 autoantibody found in patient serum by immunoassay (antibody-based) methods and thus to diagnose glioblastoma brain tumor and progression intohigh grade gliomas.

In an example of the invention, the increased expression of SLC3a2 in GB compared to control and non-GB glioma phenomena and the presence of autoantibodies produced in the human body for this protein were discovered during the invention process, and the increase of this autoantibody associated with glioma prognosis with the invention has been shown to offer an advantage especially for early diagnosis of glioblastoma.

However, by determining that SLC3a2 is an autoantigen, it may provide an early treatment option for patients with primary GB and secondary GB through increased autoantibodies in the serum. For this purpose, by means of the method of the invention, the diagnosis of GB and/or high grade glioma, therefore early treatment, is provided simply and quickly by using only the patient's blood, without undergoing surgical operation for GB patients. Apart from this, high specific targeting with the autoantibodies used in the invention may pave the way for various opportunities in the treatment of GB.

In the patent document numbered WO2015200823A1 available in the prior art, although 33 cell surface proteins identified in glioblastoma (GB) tissue are mentioned, the SLC3a2 protein of the invention and the autoantigen-autoantibody interaction are not mentioned. For this reason, it is mentioned for the first time with the invention that SLC3a2 protein has a GB-specific autoantigen interaction and that anti-SLC (especially anti-SLC3a2) autoantibodies can be used in the diagnosis and treatment methods of patients with GB and/or advanced stage glioma.

In the patent document numbered CN102590511B, it is mentioned that IGFBP-2 autoantibodies, which can be used in the diagnosis of glioblastoma along with various cancers, can be detected and used as a diagnostic kit, but its relationship with SLC3a2 autoantigen and gliomas is not mentioned. In the invention, anti-SLC3a2 autoantibody levels and the detection of different types or modified forms of autoantigen SLC3a2 in gliomas distinguish low and high grade gliomas, and it also offers a distinctive feature in the diagnosis of GB with its glioblastoma (GB) specific profile among high grade gliomas. On the other hand, since glioblastoma is the most malignant cancer, patients of which are known to be died in a short time, usually in 15 months, on average, despite treatment, the distinctive feature of GB makes anti-SLC3a2 autoantibodies significantly effective for diagnosis and treatment. In this context, it is of great importance to develop innovative approaches for developing opportunities for the treatment and early diagnosis of GB disease in terms of paving the way for GB treatment and increasing the lifetime of patients.

In the invention, it has been shown that increased levels of SLC3a2 proteins in brain tumors cause worsening of the disease and also may undergo special modifications in glioblastoma brain tumors. This makes SLC3a2 and/or its isotypes, or specific modified forms thereof, a potential biomarker for brain tumor. On the other hand, the autoantibody is a diagnostic marker that binds to this SLC3a2 and that we have shown its presence in patient serum. Therefore, SLC3a2 is a separate biomarker candidate that can be used in both diagnosis and treatment.

With these aspects, the invention provides the early diagnosis and treatment of patients with high grade glioma brain tumors by using the methods involving autoantibodies in patient sera,and theautoantigens recognized by these autoantibodies

### Definitions of Figures Explaining the Invention

**Figure 1****:** Results of Western Blot performed by using patient and control tissue/serum
**Figure 2****:** SLC3a2 staining in patient tissue sections
**Figure 3****:** Investigation of glioma grade specificity of anti-SLC3a2 autoantibodies in GB serum by IP (immunoprecipitation) method
**Figure 4****:** Expression level of SLC3a2 in GBs n=9 (GB), n=6 (non-GB), n=3 (control); p*<0.05, and p****<0.001).

### Detailed Description of the Invention

The invention is a method for evaluating the probability of having glioblastoma (GB) disease and/or advanced stage glioma brain tumor disease, it includes the process steps of
a) Determining the expression levels or presence of wild type/isoforms of SLC3a2 in tissue and/or serum samples,
b) Comparing the blood levels of autoantibodies developed in human serum samples against the protein whose level was determined in step a, and the differences between normal and glioma cases,
c) Determining whether there is an increase in the expression level of the SLC3a2 protein.

Wherein SLC3a2 proteins in their wild type and different forms can be the Solute Carrier 3a2 (SLC3a2) protein, mutant SLC3a2 protein, SLC3a2 protein isomer, modified SLC3a2, or SLC3a2 protein forming a complex with other protein or proteins.

First of all, tissue and serum samples were collected from patients with Glioblastoma (GB) and non-GB glioma, and from control subjects without tumor tissue (epilepsy patients). Protein isolation was made by homogenizing patient and control tissues. Western blot (WB) analyzes were performed by using the homogenates obtained and human serum as primary antibody and commercial HRP-conjugated anti-human IgGs as secondary antibody. As a result of WB analysis, it was observed that serum antibodies and tissue homogenates interacted significantly in the range of 30-70 kDa. Therefore, 5 controls, 5 non-GB gliomas and 5 GB tissue homogenates were analyzed by mass spectrometry to detect the candidate antigens. Samples called non-GB refer to grades I-II-III and IV gliomas without GB pathology.

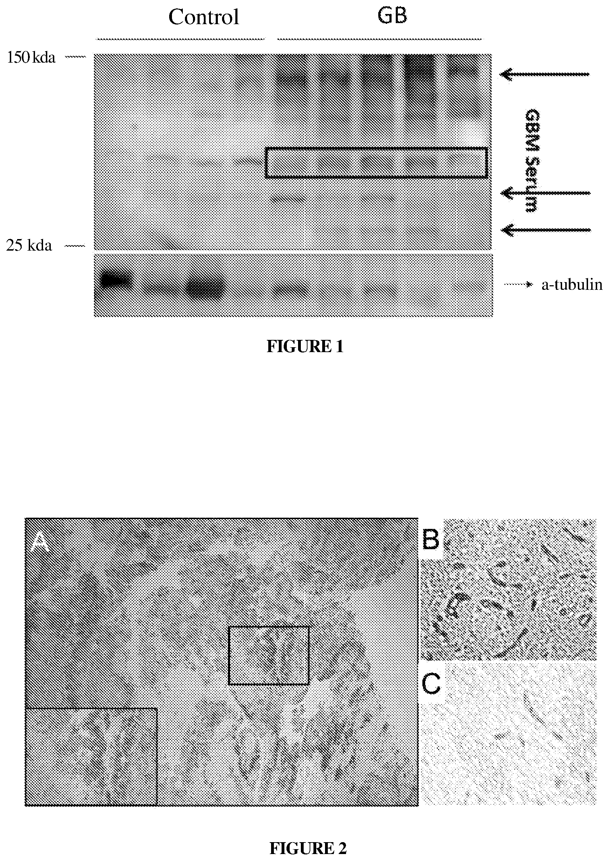

As a result of the analyses, it was observed that SLC3a2 protein, is mostly overexpressed in GBs. In addition, is has been observed that the band size of the interaction with the autoantibody, which was significant in WB analysis, is compatible with its molecular weight. Figure 1 shows WB studies performed by using patient and control tissue/serum. In this study, protein isolation was performed from glioma tissues whose grade is detected. Since healthy control brain tissue could not be obtained for the study, tissues and serum samples obtained from epilepsy patients without tumor diagnosis were used as control group. In the analyses performed using the WB method, patient/control sera were used instead of primary antibodies, and β-mercaptoethanol was not used in the sample preparation before WB as a requirement of the experimental design. The purpose of not using β-mercaptoethanol is to detect these autoantibodies by preventing their degradation by β-mercaptoethanol if the autoantibodies to be present in serum are specific to complex structures. As a result of the analyses, it was observed that GB tissues and GB sera showed a distinctive interaction pattern. Bands with distinctive interactions with GB tissues are indicated by black arrows in the figure. As a result of the analyses, protein bands with high expression levels in GB-specific/GB are detected in the range of 25-150 kda.

As a result of proteomics data and mass spectrometry analysis, a total of 3014 proteins were identified and the increased expressions of 10 of them in GB showed statistical significance. It has been detected that SLC3a2 (solute carrier 3a2) from the proteins in the kDa range in which the specific interaction was detected, is expressed 11-fold more in GB patients than in control phenomena. (p<0.001).

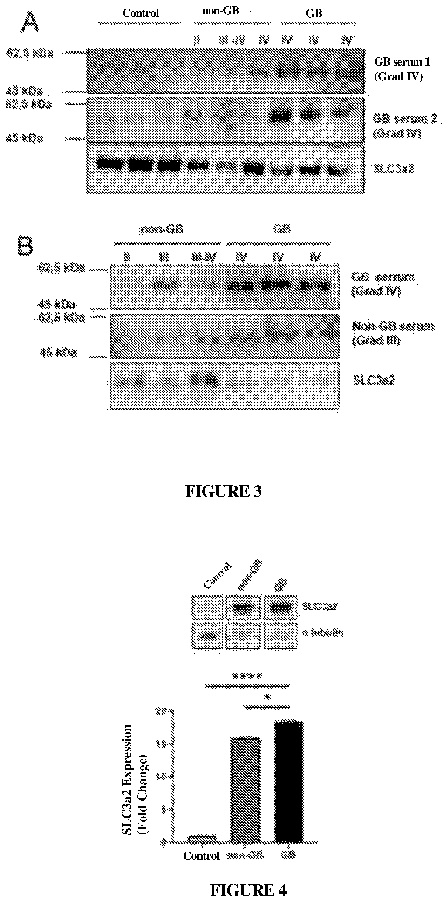

As a result of mass spectrometry analyses, a 39-fold increase was observed in SLC3a2 expression compared to control tissues. Therefore, it was investigated whether this increase was related to the band intensities in the WB analyzes performed with patient sera. WB and IHC studies were performed with control, non-GB and GB experimental groups to test whether SLC3a2 expression would be compatible with mass spectrometry results by other methods. As a result of WB analysis, it was observed that the expression of SLC3a2 showed 18.6 fold increase in GB tissues compared to the control. As seen in Figure 4, SLC3a2 shows high expression in GBs. The results of mass spectrometry were analyzed by evaluating with WB; n=9 (GB), n=6 (non-GB), n=3 (control); p*<0.05, and p****<0.001).

SLC3a2 protein was precipitated by immunoprecipitation (IP) method in 3 controls, 3 non-GB glioma and 3 GB tissue homogenates with commercial SLC3a2 antibodies and analyzed by WB method using control phenomenon, non-GB glioma and GB patient sera as primary antibodies. As a result of the analyses, the presence of autoantibodies recognizing the SLC3a2 protein expressed in GB tissue homogenates was determined in GB patient sera. Therefore, the presence of anti-SLC3a2 autoantibodies, which may be related to the prognosis of the disease and guide both the diagnosis and treatment of the disease, was detected in the serum of GB patients during the invention process, and it was shown that these autoantibodies could be used in the detection of GB. Therefore, the invention encompasses the use of autoantibodies recognizing SLC3a2 protein for the detection of GB and diagnosis methods targeting antigens (SLC proteins) recognized by these autoantibodies.

The SLC3a2 autoantibody consists of 2 heavy chains and 2 light chains and weighs approximately 150 KDa. It belongs to the immunoglobulin G family (IgG) from the antibody types. This autoantibody is present in GB tissue sera and specifically recognizes the SLC3a2 protein found in tissue homogenates of GB patients very strongly compared to non-GB glioma tissue and control tissue homogenates.

As seen in Figure 2, immunohistochemistry (IHC) stains confirm the proteomics data. SLC3a2 appears to be more expressed in GBs. In the SLC3a2 staining of patient tissue sections, intense SLC3a2 expression is observed in intracellular and cell membrane GB patient tissue sections (A) compared to non-GB Glioma (B) and epilepsy (C) tissue sections used as control phenomenon.

The fact that SLC3a2 expression was statistically significantly higher than control tissues with 3 different methods (WB, expression analysis and IHC) supports the presence of autoantibodies developed in patients against SLC3a2.

Figure 3 shows that SLC3a2 autoantibody is specific for GB sera. As a result of investigation of the specificity of anti-SLC3a2 autoantibodies in GB sera by IP method, it was analyzed with WB by using the patient with commercial SLC3a2 and patient and control sera after SLC3a2 is precipitated from patient and control group. Immunoprecipitation studies were performed to detect the presence of autoantibodies in GB sera against SLC3a2, which is overexpressed in GB tissues. For this purpose, 40 µg of control, non-GB and GB tissue lysates were precipitated with commercial SLC3a2 antibody and analyzed using GB sera as primary antibody in WB analyses. As a result of the analyses performed with various GB sera, it was observed that GB sera interacted strongly with GB tissue lysates, but weakly with non-GB and control tissue lysates so that no band could be observed. Commercial anti-SLC3a2 was used as a loading control. It was observed that only GB serum IgGs are bound very strongly to SLC3a2 specific to GB. Although non-GB and GB proteins coexist on the same membrane, SLC3a2 autoantibodies in GB sera interacted only with GB tissues. These data indicate that SLC3a2 autoantibodies in GB patient sera more strongly recognize SLC3a2 found only in GB tissues. Therefore, the data suggest that another isoform of SLC3a2 may be present in GB tissues or that SLC3a2 may be modified or mutated in GBs.

## Claims

1. A method for evaluating the probability of having glioblastoma (GB) disease and/or high grade glioma brain tumor disease, **characterized in that** it includes the process steps of;
a) Determining the expression level or presence of SLC3a2 protein in wild type and different forms in tissue and/or serum samples,
b) comparing the blood level of autoantibodies developed against the protein whose level was determined in step a, and the differences between normal and glioma phenomena
c) determining whether there is an increase in the expression level of the SLC3a2 protein.

2. A method according to claim 1, **characterized in that** SLC3a2 proteins in their wild type and different forms are the Solute Carrier 3a2 (SLC3a2) protein, mutant SLC3a2 protein, SLC3a2 protein isomer, modified SLC3a2, or SLC3a2 protein forming a complex with other protein or proteins.

3. A method according to claim 1, **characterized in that** said Solute Carrier 3a2 (SLC3a2) protein in the wild type and different forms is SLC3a2 autoantigen with which the anti-SLC3a2 autoantibody interacts.

4. A method according to claim 3, **characterized in that** Solute Carrier 3a2 (SLC3a2) protein in the wild type and different forms are the Solute Carrier 3a2 (SLC3a2) protein, mutant SLC3a2 protein, SLC3a2 protein isomer, modified SLC3a2, or SLC3a2 protein forming a complex with other protein or proteins.

5. A method according to claim 1, **characterized in that** the antibodies are bound to said Solute Carrier 3a2 (SLC3a2) proteins.

## Patentansprüche

1. Ein Verfahren zur Bewertung der Wahrscheinlichkeit, an einer Glioblastom (GB) Erkrankung und/oder einem hochgradigen Gliom-Hirntumor zu erkranken, **dadurch gekennzeichnet, dass** es die folgenden Prozessschritte umfasst:
a) Bestimmung des Expressionspiegel oder des Vorhandenseins des SLC3a2-Proteins im Wildtyp und in unterschiedlichen Formen in Gewebe- und/oder Serumproben,
b) Vergleich des Blutspiegels der Autoantikörper, die sich gegen das Protein entwickelt haben, dessen Spiegel in Schritt a bestimmt wurde, und der Unterschiede zwischen normalen und Gliomphänomenen
c) Bestimmung, ob es zu einem Anstieg des Expressionspiegel des SLC3a2-Proteins kommt.

2. Verfahren nach Anspruch 1, **dadurch gekennzeichnet, dass** es sich bei den SLC3a2-Proteinen in ihrem Wildtyp und unterschiedlichen Formen um das Solute Carrier 3a2 (SLC3a2)-Protein, das mutierte SLC3a2-Protein, das SLC3a2-Proteinisomer, das modifizierte SLC3a2 oder das SLC3a2-Protein handelt, das einen Komplex mit einem oder mehreren anderen Proteinen bildet.

3. Verfahren nach Anspruch 1, **dadurch gekennzeichnet, dass** das Solute Carrier 3a2 (SLC3a2)-Protein im Wildtyp und in unterschiedlichen Formen das SLC3a2-Autoantigen ist, mit dem der Anti-SLC3a2-Autoantikörper interagiert.

4. Verfahren nach Anspruch 3, **dadurch gekennzeichnet, dass** das Solute Carrier 3a2 (SLC3a2)-Protein im Wildtyp und in unterschiedlichen Formen das Solute Carrier 3a2 (SLC3a2)-Protein, das mutierte SLC3a2-Protein, das SLC3a2-Proteinisomer, das modifizierte SLC3a2 oder das SLC3a2-Protein ist, das einen Komplex mit einem oder mehreren anderen Proteinen bildet.

5. Verfahren nach Anspruch 1, **dadurch gekennzeichnet, dass** die Antikörper an die Solute Carrier 3a2 (SLC3a2)-Proteine gebunden sind.

## Revendications

1. Méthode d'évaluation de la probabilité de souffrir de la maladie du glioblastome (GB) et/ou d'une tumeur au cerveau de gliome de haut grade, **caractérisée en ce que** le processus comprend les étapes suivantes ;
a) déterminer le niveau d'expression ou la présence de la protéine SLC3a2 de type sauvage et différentes formes dans des échantillons de tissus et/ou de sérum,
b) comparer le niveau sanguin des autoanticorps développés contre la protéine dont le niveau a été déterminé à l'étape a, et les différences entre les phénomènes normaux et de gliome,
c) déterminer s'il y a une augmentation du niveau d'expression de la protéine SLC3a2.

2. Méthode selon la revendication 1, **caractérisée en ce que** les protéines SLC3a2 de type sauvage et différentes formes sont la protéine de Transporteur de Soluté 3a2 (SLC3a2), la protéine SLC3a2 mutante, l'isomère de la protéine SLC3a2, la SLC3a2 modifiée ou la protéine SLC3a2 formant un complexe avec une autre protéine ou d'autres protéines.

3. Méthode selon la revendication 1, **caractérisée en ce que** ladite protéine de Transporteur de Soluté 3a2 (SLC3a2) de type sauvage et différentes formes est l'autoantigène SLC3a2 avec lequel l'autoanticorps anti-SLC3a2 interagit.

4. Méthode selon la revendication 3, **caractérisée en ce que** la protéine de Transporteur de Soluté 3a2 (SLC3a2) de type sauvage et différentes formes sont la protéine de Transporteur de Soluté 3a2 (SLC3a2), la protéine SLC3a2 mutante, l'isomère de la protéine SLC3a2, la SLC3a2 modifiée ou la protéine SLC3a2 formant un complexe avec une autre protéine ou d'autres protéines.

5. Méthode selon la revendication 1, **caractérisée en ce que** les anticorps sont liés auxdites protéines de Transporteur de Soluté 3a2 (SLC3a2).
